(19) Europäisches Patentamt
European Patent Office
Office européen des brevets

(11) EP 4 257 205 A2

(12) **EUROPEAN PATENT APPLICATION**

(43) Date of publication:
**11.10.2023 Bulletin 2023/41**

(21) Application number: **23195420.7**

(22) Date of filing: **20.01.2017**

(51) International Patent Classification (IPC):
***A61P 43/00*** *(2006.01)*

(52) Cooperative Patent Classification (CPC):
**A61K 31/519; A61K 9/06; A61P 17/00; A61P 17/04; A61P 43/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.01.2016 EP 16152215**

(62) Document number(s) of the earlier application(s) in accordance with Art. 76 EPC:
**17701828.0 / 3 405 197**

(71) Applicant: **LEO Pharma A/S**
**2750 Ballerup (DK)**

(72) Inventors:
- **WENNBERG, Tero**
  **2750 Ballerup (DK)**
- **SØRENSEN, Anders Per**
  **3500 Værløse (DK)**

(74) Representative: **Zacco Denmark A/S**
**Arne Jacobsens Allé 15**
**2300 Copenhagen S (DK)**

Remarks:
This application was filed on 05-09-2023 as a divisional application to the application mentioned under INID code 62.

(54) **TREATMENT OF HAND ECZEMA**

(57) The present invention relates to the treatment of hand eczema.The problem to be solved by the invention is to provide a new pharmaceutical use of 3-[(3S,4R)-3-methyl-6-(7H-pyrrolo[2,3-d]pyrimidin-4-yl)-1,6-diazaspiro[3.4]octan-1-yl]-3-oxopropanenitrile. A therapeutic or preventive agent for hand eczema, containing 3-[(3S,4R)-3-methyl-6-(7H-pyrrolo[2,3-d]pyrimidin-4-yl)-1,6-diazaspiro[3.4]octan-1-yl]-3-oxopropanenitrile as an active ingredient.

EP 4 257 205 A2

## Description

FIELD OF THE INVENTION

The present invention relates to a new pharmaceutical use of 3-[(3S,4R)-3-methyl-6-(7H-pyrrolo[2,3-d]pyrimidin-4-yl)-1,6-diazaspiro[3.4]octan-1-yl]-3-oxopropanenitrile. In another aspect, the present invention relates to the treatment of hand eczema.

BACKGROUND OF THE INVENTION

Hand eczema is an inflammatory skin disorder, clinically characterized by erythema, infiltration, hyperkeratosis, oedema, and vesicles. Secondary signs include scaling, fissures, erosions and bacterial infections.
Management of hand eczema involves identification and elimination of allergic or irritant triggers, general skin care with emollients and barrier creams, treatment of secondary skin infections, and anti-inflammatory therapies. Topically applied corticosteroids remain the mainstay of anti-inflammatory therapy for hand eczema, but long-term use is restricted due to side effects.
Non-steroidal treatments include topically applied calcineurin inhibitors, Grenz rays and phototherapy.
Systemic immunosuppressive therapies are used for severe hand eczema, but have the potential of severe, systemic adverse events, which limits their use.
Several JAK-inhibitors are in clinical development or are already on the market. Ruxolitinib, the first FDA approved JAK1 and JAK2 inhibitor, is an oral drug which is approved in several countries/regions for treatment of patients with myelofibrosis. Tofacitinib is currently approved in the US as an oral drug for treatment of rheumatoid arthritis and is currently under development for treatment of psoriasis and atopic dermatitis.
WO2011/013785 describes nitrogen-containing spirocyclic compounds and pharmaceutical uses thereof. The compounds are stated to be JAK kinase 3 inhibitors useful for the prevention or treatment of e.g. autoimmune diseases, allergic diseases, psoriasis, rheumatoid arthritis and atopic dermatitis.
EP 2813228 A1 describes the pharmaceutical use of a JAK inhibitor, and more specifically a pharmaceutical composition for treating skin diseases such as senile xerosis, asteatosis, eczema and contact dermatitis.
Consequently, there is an unmet medical need for new treatment of hand eczema with high efficacy in combination with an attractive safety profile.

SUMMARY OF THE INVENTION

The present invention relates to the treatment of hand eczema comprising the compound of formula (I)

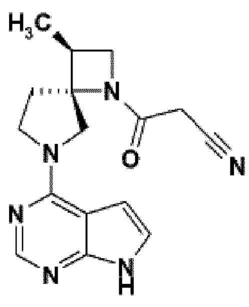

(I),

3-[(3S,4R)-3-methyl-6-(7H-pyrrolo[2,3d]pyrimidin-4-yl)-1,6-diazaspiro[3,4]octan-1-yl]-3-oxopropanenitrile, or a pharmaceutically acceptable salt thereof.
In one aspect the present invention relates to the use of the compound of formula (I) for use in the treatment of hand eczema.
In another aspect, the present invention relates to the compound of formula (I) for use in the treatment of hand eczema. In yet another aspect, the invention relates to the compound of formula (I) for use in topical treatment of hand eczema. In yet another aspect, the topical formulation is an ointment. In yet another aspect, the present invention relates to the use of the compound of formula (I), which is administered twice daily for 8 weeks. In another aspect, the present invention relates to the compound of formula (I) which is administered in a concentration of 30 mg/g. In yet another aspect, the compound of formula (I) is administered in an amount of 1.7 $mg/cm^2$.
In another aspect, the present invention relates to the use of the compound of formula (I) in the manufacture of a pharmaceutical composition for the treatment of hand eczema. In yet another aspect, the pharmaceutical composition

is a topical formulation. In yet another aspect the topical formulation is an ointment.

In another aspect, the present invention relates to a pharmaceutical composition comprising a compound of formula (I)

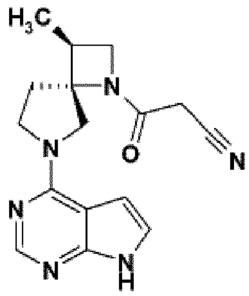

(I),

3-[(3S,4R)-3-methyl-6-(7H-pyrrolo[2,3d]pyrimidin-4-yl)-1,6-diazaspiro[3,4]octan-1-yl]-3-oxopropanenitrile, or a pharmaceutically acceptable salt thereof, for the treatment of hand eczema.

In another aspect, the present invention relates to the pharmaceutical composition comprising the compound of formula (I) wherein the treatment is topical, e.g. treatment with an ointment.

In another aspect, the present invention relates to the pharmaceutical composition wherein the compound of formula (I) is administered as 30 mg/g concentration. In another aspect, the compound of formula (I) is administered as a twice daily application for 8 weeks.

In another aspect, the present invention relates to the pharmaceutical composition wherein the compound of formula (I) is administered in an amount of 1.7 $mg/cm^2$.

In another aspect, the present invention relates to a method for treating hand eczema in a subject in need thereof, which method comprises the step of administering to said subject a therapeutically effective amount of the compound of formula (I).

In another aspect, the present invention relates to a method for treating hand eczema in a subject in need thereof, wherein the administration is topical.

In another aspect, the present invention relates to a method for treating hand eczema in a subject in need thereof, wherein the topical formulation is an ointment.

In another aspect, the present invention relates to a method for treating hand eczema in a subject in need thereof, wherein the compound of formula (I) is administered in a concentration of 30 mg/g.

The present invention also provides for the use of 3-[(3S,4R)-3-methyl-6-(7H-pyrrolo[2,3-d]pyrimidin-4-yl)-1,6-diazaspiro[3,4]octan-1-yl]-3-oxopropanenitrile in the treatment of hand eczema.

The present invention further provides for the above use administered as a twice daily application.

The present invention further provides for the above use administered in a concentration of 30 mg/g.

BRIEF DESCRIPTION OF THE DRAWING

Fig. 1 A chart illustrating the "treatment success" according to Physician's Global Assessment of disease severity (PGA) in a clinical trial comparing the efficacy of twice daily application of the compound of formula (I) in ointment 30 mg/g with ointment vehicle for up to 8 weeks in treatment of subjects with hand eczema.

DETAILED DESCRIPTION OF THE INVENTION

The compound of formula (I)

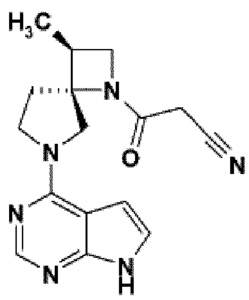

(I),

3-[(3S,4R)-3-methyl-6-(7H-pyrrolo[2,3d]pyrimidin-4-yl)-1,6-diazaspiro[3,4]octan-1-yl]-3-oxopropanenitrile, was described previously in WO2011/013785 as a JAK 3 kinase inhibitor for the treatment of e.g. autoimmune diseases, allergic diseases, psoriasis, rheumatoid arthritis and atopic dermatitis, and in EP 2813228 A1 for treating skin diseases such as senile xerosis, asteatosis, eczema and contact dermatitis.

The compound of formula (I) can be produced according to the method described in Preparation 6 of WO2011/013785.

The "pharmaceutically acceptable salt" may be any non-toxic salts of the compound of formula (I), and includes a salt with an inorganic acid, a salt with an organic acid, a salt with an inorganic base, a salt with an organic base, and a salt with an amino acid.

The salt with an inorganic acid includes a salt with hydrochloric acid, nitric acid, sulphuric acid,hosphoriccid, hydrobromic acid, etc. The salt with an organic acid includes salt with oxalic acid, maleic acid, citric acid, fumaric acid, lactic acid, malic acid, succinic acid, tartaric acid, acetic acid, trifluoroacetic acid, gluconic acid, ascorbic acid, methanesulfonic acid, benzenesulfonicacid, p-toluenesulfonic acid, etc. The salt with an inorganic base includes sodium salt, potassium salt, calcium salt, magnesium salt, ammonium salt, etc. The salt with an organic base includes a salt with methylamine, diethylamine, trimethylamine, triethylamine, ethanolamine, diethanolamine, triethanolamine, ethylenediamine, tris(hydroxymethyl)methylamine, dicyclohexylamine, N,N'-dibenzylethylenediamine, guanidine, pyridine, picoline, choline, cinchonine, meglumine, etc. The salt with an amino acid includes a salt with lysine, arginine, aspartic acid, glutamic acid, etc.

According to well-known methods, each salt may be obtained by reacting the compound of formula (I) with an inorganic base, an organic base, an inorganic acid, an organic acid or an amino acid.

The compound of formula (I) may be labeled with isotopes, e.g. $^{3}H$, $^{14}C$, $^{35}S$.

In one aspect, the compound of formula (I) or a pharmaceutically acceptable salt thereof is the substantially purified compound of formula (I) or a pharmaceutically acceptable salt thereof. In another aspect the compound of formula (I) or a pharmaceutically acceptable salt thereof is in the purity of 80% or more.

The "pharmaceutical composition" includes an oral preparation such as tablet, capsule, granule, powder, lozenge, syrup, emulsion and suspension, or a parenteral preparation such as topical agent, suppository, injection, eyedrop, nasal drug and pulmonary drug. In another aspect, the pharmaceutical composition is a topical agent. In yet another aspect, the pharmaceutical composition is an ointment.

The pharmaceutical composition of the present invention is produced by appropriately mixing the compound of formula (I) or a pharmaceutically acceptable salt thereof with at least one type of pharmaceutically acceptable carriers in appropriate amounts according to methods known in the technical field of medicinal preparation. Thecontent of the compound of formula (I) or a pharmaceutically acceptable salt thereof in the pharmaceutical composition depends on its dosage forms, dosage amounts, etc., and for example, is 0.1 to 100% by weight of the entire composition. For example, the content is 0.25, 0.30, 0.50, 0.75, 1.0,1.25,1.50, 1.75, 2.00, 2.25, 2.50, 2.75, 3.00, 3.25, 3.50, 3.75 or 4.00% by weight of the entire composition. In another aspect, the content of the compound of formula (I) or a pharmaceutically acceptable salt thereof in the pharmaceutical composition is 3% by weight of the entire composition.

The term "therapeutically effective amount" of the compound as used herein means an amount sufficient to cure, alleviate or partially arrest the clinical manifestations of a given disease and its complications. An amount adequate to accomplish this is defined as "therapeutically effective amount". Effective amounts for each purpose will depend on the severity of the disease or injury as well as the weight and general state of the subject.

The "pharmaceutically acceptable carrier" includes various conventional organic or inorganic carrier substances for pharmaceutical materials, e.g., an excipient, a disintegrant, a binder, a fluidizer, and a lubricant for solid preparations, a solvent, a solubilizing agent, a suspending agent, a tonicity agent, a buffer, and a soothing agent for liquid preparations, and a base, an emulsifier, a humectant, astabilizer,astabilizing agent, a dispersant, a plasticizer, a pH regulator, an absorption promoter, a gelling agent, an antiseptic, a filler, a resolvent, a solubilizing agent, and a suspending agent for semisolid preparations. Further, an additive including a preserving agent, an antioxidant agent, and a colorant may be used, if needed.

The "excipient" includes lactose, white soft sugar, D-mannitol, D-sorbitol, cornstarch, dextrin, microcrystalline cellulose, crystalline cellulose, carmellose, carmellose calcium, sodium carboxymethyl starch, low-substituted hydroxypropyl cellulose, gum arabic, etc.

The "disintegrant" includes carmellose, carmellose calcium, carmellose sodium, sodium carboxymethyl starch, croscarmellose sodium, crospovidone, low-substituted hydroxypropyl cellulose, hydroxypropyl methylcellulose, crystalline cellulose, etc.

The "binder" includes hydroxypropyl cellulose, hydroxypropyl methylcellulose, povidone, crystalline cellulose, white soft sugar, dextrin, starch, gelatin, carmellose sodium, gum arabic, etc.

The "fluidizer" includes light anhydrous silicic acid, magnesium stearate, etc.

The "lubricant" includes magnesium stearate, calcium stearate, talc, etc.

The "solvent" includes purified water, ethanol, propylene glycol, macrogol, sesame oil, corn oil, olive oil, etc.

The "solubilizing agent" includes propylene glycol, D-mannitol, benzylbenzoate, ethanol, triethanolamine, sodium carbonate, sodium citrate, etc.

The "suspending agent" includes benzalkonium chloride, carmellose, hydroxypropyl cellulose, propylene glycol, povidone, methyl cellulose, glyceryl monostearate, etc.

The "tonicity agent" includes glucose, D-sorbitol, sodium chloride, D-mannitol, etc.

The"buffer" includes sodium hydrogen phosphate, sodium acetate, sodium carbonate, sodium citrate, etc.

The "soothing agent" includes benzyl alcohol, etc.

The "base" includes water, animal or plant oils (e.g., olive oil, corn oil, peanut oil, sesame oil, castor oil, squalane, etc.), lower alcohols (e.g., ethanol, propanol, propylene glycol, 1,3-butylene glycol, phenol, etc.), higher fatty acids and esters thereof, waxes, higher alcohols, polyhydricalcohols, hydrocarbons (e.g., white soft paraffin, liquid paraffin, paraffin, hard paraffin, etc.), hydrophilic petrolatum, purified lanolin, absorptive ointment, hydrous lanolin, hydrophilic ointment, starch, pullulan, gum arabic, tragacanth gum, gelatin, dextran, cellulose derivatives (e.g., methyl cellulose, carboxymethyl cellulose, hydroxyethyl (e.g., carboxyvinyl polymer, sodium polyacrylate, polyvinyl alcohol, polyvinyl pyrrolidone, etc.), propylene glycol, macrogol (e.g., macrogol 200 to 600, etc.), and combinations of two or more kinds of these. In one aspect, the base is a combination of white soft paraffin, hard paraffin and squalane.

The "preserving agent" includes ethyl parahydroxybenzoate, chlorobutanol, benzyl alcohol, sodium dehydroacetate, sorbic acid, etc.

The "antioxidant agent" includes sodium sulfite, ascorbic acid, etc.

The "colorant" includes food dye (e.g., Food Red No. 2 or No. 3, Food Yellow No. 4 or No. 5, etc.), β-carotene, etc.

In one aspect, the carrier is a combination of white soft paraffin, hard paraffin and squalane. White soft paraffin, hard paraffin and squalane may be combined at a blend ratio of 70 to 90% by weight, 5 to 10% by weight and 5 to 20% by weight, respectively. In one aspect a preparation may contain the compound of formula (I), white soft paraffin, 5 $\pm$ 2% by weight of hard paraffin and 10 $\pm$ 2% by weight of squalane.

The pharmaceutical composition of the present invention may be administered to a mammal, such as a human being, a mouse, a rat, a hamster, a guinea pig, a rabbit, a cat, a dog, a pig, a cow, a horse, sheep, a monkey. In one aspect, the mammal is a human being. A dosage amount depends on subjects, diseases, symptoms, dosages forms, administration routes, etc., and may be in the range from about 0.01 mg to1 g per day in terms of the content of the compound of formula (I) as an active ingredient. The dose may be administered at a time or in several divided doses.

A topical agent may be applied, for example, by application, inunction or spraying depending on the dosage form, etc. An application amount of the topical agent to the affected area can be selected depending on the content of the active ingredient, etc., and the topical agent can be applied, for example, at a time or in several divided amounts per day. The preferable application is once daily or twice daily.

The phrase "JAK" refers to one or more enzymes of JAK1, JAK2, JAK3, and TYK2 belonging to JAK family.

The phrase "inhibitJAK" refers to inhibiting functions ofJAK to disappear or attenuate its activity, and inhibiting one or more enzymes belonging to JAK family. In one aspect, the phrase "inhibit JAK" refers to "inhibit human JAK". The inhibition of functions or the disappearance or attenuation of the activity is, in one aspect, conducted in the situations of human clinical application.

The "JAK inhibitor" may be any substances which inhibit JAK, and may be, for example, low-molecular weight compounds, nucleic acid, polypeptide, protein, antibody, vaccine, etc. In one aspect, the "JAK inhibitor" is a "human JAK inhibitor". In another aspect, the JAK inhibitor is the compound of formula (I) or a pharmaceutically acceptable salt thereof. In yet another aspect, the JAK inhibitor is the compound of formula (I).

The term "treatment" as used herein includes amelioration of a symptom, prevention of an aggravation, maintenance of a remission, prevention of an exacerbation, and prevention of a recurrence. The term "prevention" refers to suppressing occurrence of a symptom.

The term "treatment" may also include the delaying of the progression of the disease, disorder or condition, the amelioration, alleviation or relief of symptoms and complications, and/or cure or elimination of the disease, disorder or condition. The term "treatment" may also mean the management and care of a patient for the purpose of combating the disease, condition or disorder.

The terms "disease", "disorder" and "condition" as used herein are used interchangeably to specify a state of a patient which is not the normal physiological state of a human being.

The term "hand eczema" may also include chronic hand eczema, where chronic hand eczema is that which persists more than months or returns twice or more within 12 months despite adequate dermatological therapy and patient compliance.

An embodiment of the present invention includes a pharmaceutical composition for treating or preventing hand eczema, comprising 3-[(3S,4R)-3-methyl-6-(7H-pyrrolo[2,3-d]pyrimidin-4-yl)-1,6-diazaspiro[3.4]octan-1-yl]-3-oxopropanenitrile and a pharmaceutically acceptable carrier.

An embodiment of the present invention includes a use of 3-[(3S,4R)-3-methyl-6-(7H-pyrrolo[2,3-d]pyrimidin-4-yl)-1,6-diazaspiro[3.4]octan-1-yl]-3-oxopropanenitrile for treating or preventing hand eczema.

An embodiment of the present invention includes a method for treating or preventing hand eczema, characterized by administering to a mammal a therapeutically effective amount of 3-[(3S,4R)-3-methyl-6-(7H-pyrrolo[2,3-d]pyrimidin-4-yl)-1,6-diazaspiro-[3.4]octan-1-yl]-3-oxopropanenitrile.

The mammal is a human being, a mouse, a rat, a hamster, a guinea pig, a rabbit, a cat, a dog, a pig, a cow, a horse, sheep, a monkey. In one aspect the mammal is a human being.

In another aspect, the human being is a person suffering from a disease who is in need of medical care.

In another aspect, the present invention relates to a therapeutic or preventive agent for hand eczema, comprising the compound of formula (I).

In another aspect, the present invention relates to a pharmaceutical composition, comprising the compound of formula (I) and a pharmaceutically acceptable carrier.

The present invention describes its effect in topical treatment of hand eczema.

The invention provides a clinical trial to compare efficacy of twice daily topical ointment with 30 mg/g of the compound of formula (I), with an ointment vehicle for 8 weeks in the treatment of hand eczema.

The compound of formula (I) is formulated in an ointment containing the active compound in 30 mg/g, white soft paraffin, hard paraffin and squalane.

Dosis range will be a thin layer covering the affected areas on the hands corresponding to 1.7 mg/cm$^2$ of the compound of formula (I).

Depending on the size of the affected areas on the hands a maximum of 760 cm$^2$ (4% body surface area) could be treated corresponding to a maximum of 1,3 g per application.

Main assessments

The primary efficacy parameter for treatment success' is Physician's Global Assessment of disease severity (PGA).

Hand Eczema Severity Index (HECSI) and Patient's Global Assessment of disease severity (PaGA) serve as secondary parameter.

Primary efficacy parameter

"Treatment success" according to the Physician's Global Assessment of disease severity (PGA) is defined as at Visit (6) (End of Treatment):

- Subjects classified at baseline as having "mild" disease according to PGA must achieve "clear" to be considered to have achieved "treatment success".
- Subjects classified at baseline as having "moderate" or "severe" disease according to PGA must achieve "clear" or "almost clear" to be considered to have achieved "treatment success".

Secondary parameter

- The Hand Eczema Severity Index (HECSI) at Visit 6 (End of Treatment).

- Subjects with "treatment success" according to the Patient's Global Assessment of disease severity (PaGA) at Visit 6 (End of Treatment).

"Treatment success" according to the PaGA is defined as:

- Subjects classified at baseline as having "'very mild" or "mild" disease according to PaGA must achieve "clear" to be considered to have achieved "treatment success".

- Subjects classified at baseline as having "moderate" or "severe" disease according to PaGA must achieve "clear" or "very mild" to be considered to have achieved "treatment success".

Physician's Global Assessment of disease severity (PGA)

PGA is using the following 5 point scale as shown in Table I. The assessment is based on the condition of the disease at the time of the evaluation and not in relation to the condition at a previous visit.

Table I Physician's Global Assessment of disease severity (PGA)

| PGA | | | |
|---|---|---|---|
| PGA Severity | Features | Intensity | Area involved* |
| Clear | Erythema, scaling, hyperkeratosis/ lichenification<br>Vesiculation, oedema, fissures, puritus/ pain | absent<br>absent | Not detectable |
| Almost clear | Erythema, scaling, hyperkeratosis/ lichenification<br>Vesiculation, oedema, fissures, puritus/ pain | at least one mild<br>absent | Less than 10% of affected hand surface |
| Mild | Erythema, scaling, hyperkeratosis/ lichenification<br>Vesiculation, oedema, fissures, puritus/ pain | at least one mild<br>at least one mild | Less than 10% of affected hand surface |
| Moderate | Erythema, scaling, hyperkeratosis/ lichenification | at least one mild or moderate | 10-30% of affected hand |
| | Vesiculation, oedema, fissures, puritus/ pain | at least one moderate | surface |
| Severe | Erythema, scaling, hyperkeratosis/ lichenification<br>Vesiculation, oedema, fissures, puritus/ pain | at least one moderate or<br>at least one severe | > 30% of affected hand surface |

The severity of each PGA sign or symptom is described in Table II

Table II Description of the severity of each PGA Sion or Symptom

| Parameter | | Description of severity |
|---|---|---|
| Erythema | mild<br>moderate<br>severe | faint erythema<br>prominent redness<br>deep intense red colour |
| Scaling | mild<br>moderate<br>severe | slight flaking over limited areas, mostly fine scales<br>flaking over widespread area(s), coarser scales<br>desquamation covering over 30% of the hand, with coarse thick scales |
| Lichenification/ hyperkeratosis | mild<br>moderate<br>severe | mild thickening with exaggerated skin lines over limited areas<br>palpable thickening over widespread area(s)<br>prominent thickening over widespread area (s) with exaggeration of normal skin markings |
| Vesiculation | mild<br>moderate<br>severe | scattered vesicles affecting up to 10% of hand, without erosion<br>scattered or clustered vesicles affecting up to 30% of hand, without visible erosion or excoriation<br>high density of vesicles extending over large area(s), or with erosion or excoriation |
| Oedema | mild<br>moderate<br>severe | dermal swelling over less than 10% of hands<br>definite dermal swelling over more than 10% of hand<br>dermal swelling with skin induration over widespread area(s) |

(continued)

| Parameter | | Description of severity |
|---|---|---|
| Fissures | mild<br>moderate<br><br>severe | cracked skin affecting a small area of the hand<br>cracked skin affecting multiple areas of the hand and causing pain<br>one or more deep fissures and causing bleeding or severe pain |
| Pruritus/pain | mild<br>moderate<br>severe | occasional, slight discomfort frequently during the day<br>intermittent, causing discomfort frequently during the day<br>persistent or interfering with sleep |

<u>The Hand Eczema Severity Index (HECSI)</u>

HECSI to be assessed in this clinical trial is a composite score evaluating the severity of the 6 clinical signs (erythema (E), infiltration/papulation (I), vesicles (V), fissures (F), scaling (S), oedema (O)) and the extent of the lesions on each of the 5 hand areas (fingertips, fingers (except tips), palms, back of hands and wrists) by use of standard scales.

For each hand area (total of both hands e.g. 10 fingers), the (sub)investigator rates the average severity of each of the 6 clinical signs of hand eczema using the following 4 point severity scale:

| | | |
|---|---|---|
| 0 | = | none/absent |
| 1 | = | mild |
| 2 | = | moderate |
| 3 | = | severe |

Intermediate ratings (i.e. 0.5, 1.5, 2.5) are not permitted.

For each hand area, the (sub)investigator also rates the extent of the lesions by assessing the percentage of the areas these lesions occupy, and converting it to a score based on a 5-point scale (the affected area score [AS]) as follows:

| | | |
|---|---|---|
| 0 | = | 0% affected area |
| 1 | = | 1% to 25% affected area |
| 2 | = | 26% to 50% affected area |
| 3 | = | 51% to 75% affected area |
| 4 | = | 76% to 100% affected area |

For each of the hand areas, the grades for 6 selected clinical signs of hand eczema are totalled (E) + (I) + (V) + (F) + (S) + (O) = total grade (TG)) and this TG will be multiplied by the converted affected AS.

The total HECSI score is calculated as follows:

| Hand Region | HECSI Score |
|---|---|
| Fingertips (FT) | (E+I+V+F+S+O) x AS |
| Finger (F) | (E+I+V+F+S+O) x AS |
| Palms (P) | (E+I+V+F+S+O) x AS |
| Back of Hands (BH) | (E+I+V+F+S+O) x AS |
| Wrists (W) | (E+I+V+F+S+O) x AS |
| HECSI = | Sum of the above 5 region scores |

The highest possible HECSI score is 360.

Patient's Global Assessment of disease severity (PaGA)

PaGA a self-assessment of overall disease severity using the below 5 point scale.

Table III Patient's global assessment of disease severity (PaGA)

| Scale | Level | Definition |
|---|---|---|
| 0 | Clear | no symptoms of hand eczema at all |
| 1 | Very mild | very slight symptoms of hand eczema, does not interfere with daily life |
| 2 | Mild | slight symptoms of hand eczema, interferes with daily life only occasionally |
| 3 | Moderate | definite symptoms of hand eczema, interferes with daily life frequently |
| 4 | Severe | intense symptoms of hand eczema, interferes or restricts daily life very frequently. |

EXAMPLES

Clinical trial

The clinical trial was a multi-center, prospective, randomized, double-blind, 2 arms, vehicle controlled, parallel group, phase 2a trial in subjects with chronic hand eczema with twice daily topical application of the compound of formula (I) ointment 30 mg/g or the compound of formula (I) ointment vehicle for 8 weeks.

90 male or female subjects were randomized in the clinical trial. Randomization was in a 2:1 ratio for the active ingredient and stratified by predominant disease type (irritant, non-irritant). A cap was introduced to prevent randomization of more than 45 subjects in the irritant strata.

During the treatment phase subjects were required to return to the trial sites on Visit 2 (Week 1), Visit 3 (Week 2), Visit 4 (Week 4), Visit 5 (Week 6) and Visit 6 (Week 8, End of Treatment). A phone call with the subjects to inquire adverse events including application site reactions and potential application questions was performed on Day 3.

Clinical assessment (Physician's Global Assessment of disease severity (PGA) and Hand Eczema Severity Index (HECSI)) was undertaken at Visit 1 (baseline) and after 1, 2, 4, 6 and 8 (End of Treatment) weeks of treatment. Patient's Global Assessment of disease severity (PaGA) was recorded at the same visits.

Inclusion criteria:

1. Male and female patients aged 18 - 65 years with chronic hand eczema.
2. Clinical diagnosis of chronic hand eczema with or without atopic etiology/background with a history of not adequately controlled disease activity with topically applied steroid.
3. Physician's Global Assessment of disease severity graded as at least mild at Visit 1.
4. In overall good health including well controlled diseases (e.g. hypertension, diabetes and thyroid disease) as determined by medical history, physical examination, electrocardiogram (ECG), vital signs (blood pressure, heart rate and body temperature) and clinical laboratory evaluation.

Exclusion criteria:

1. Systemic treatment with immunosuppressive drugs (e.g. methotrexate, cyclosporine, azathioprine), retinoids (e.g. alitretionin) or corticosteroids within 6 weeks prior to randomization (inhaled or intra-nasal steroids corresponding to up to 1 mg prednisone for asthma or rhinitis may be used).
2. PUVA or UVB therapy on the hands within 4 weeks prior to randomization.
3. topically applied treatment with immunomodulators (pimecrolimus, tacrolimus) or corticosteroids - on the hands within 2 weeks prior to randomization.
4. Other topically applied therapy on the hands (except for the use of subject's own emollients) within 1 week prior to randomization.

5. Use of other treatment (drug, non-drug) on the hands during the trial except for the use of IP and subject's own emollient or new emollient provided by sponsor during the clinical trial.
6. Use of systemic antibiotics or topically applied antibiotics on the hands within 2 weeks prior to randomization.
7. New topically applied treatments in regions other than the hands which could interfere with clinical trial evaluations or pose a safety concern within 1 week prior to randomization.
8. Change in systemic antihistamine therapy within two weeks prior to randomization, i.e. the subjects should not start antihistamine treatment or change current dosage regime within 2 weeks prior to randomization.
9. Concurrent skin diseases on the hands.
10. Current diagnosis of exfoliative dermatitis.
11. Significant clinical infection (impetiginized hand eczema) on the hands which requires antibiotic treatment.

Investigational product (IP)

Product: the compound of formula (I) ointment 30 mg/g for topical application:

| Component | Amount (mg/g) |
|---|---|
| the compound of formula (I) | 30 |
| Paraffin, white soft | 820 |
| Paraffin, hard | 50 |
| Squalane | 100 |

Composition of the compound of formula (I) ointment Vehicle:

| Component | Amount (mg/g) |
|---|---|
| Sunset Yellow FCF aluminium lake (color added to the vehicle to match the color of Compound A ointment 30mg/g) | 0.041 |
| Paraffin, white soft | 850 |
| Paraffin, hard | 50 |
| Squalane | 100 |

Results

The primary objective, to compare the efficacy of twice daily application of the compound of formula (I) in ointment 30 mg/g with ointment vehicle for up to 8 weeks in treatment of subjects with hand eczema resulted in "treatment success", according to Physician's Global Assessment of disease severity (PGA). In PGA, "treatment success" is defined as Subjects achieving "clear" or "almost clear" with at least a two step reduction from baseline.
The prespecified analysis comparing the two treatments at week 8 came out with a statisticallly significant (P=0.009) difference in favor of the compound of formula (I).
The results are shown in Fig 1.
Further, the study showed that the administration of the compound of formula (I) in ointment 30 mg/g was safe and well tolerated in patients with hand eczema.

CLAUSES

In view of the description, the present invention has in particular provided:

Clause 1. A compound of general formula (I)

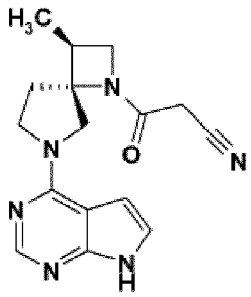

(I)

3-[(3S,4R)-3-methyl-6-(7H-pyrrolo[2,3d]pyrimidin-4-yl)-1,6-diazaspiro[3,4]octan-1-yl]-3-oxopropanenitrile, or a pharmaceutically acceptable salt thereof, for use in the treatment of hand eczema.

Clause 2. The compound according to clause 1, for use in the treatment of chronic hand eczema.

Clause 3. The compound according to clause 1 or 2 wherein the treatment is topical treatment.

Clause 4. The compound according to any one of the preceding clauses wherein the compound of formula (I), or a pharmaceutically acceptable salt thereof, is administered in an ointment.

Clause 5. The compound according to any one of the preceding clauses wherein the compound of formula (I), or a pharmaceutically acceptable salt thereof, is administered as 30 mg/g concentration.

Clause 6. The compound according to any one of the preceding clauses wherein the compound of formula (I), or a pharmaceutically acceptable salt thereof, is administered as a twice daily application.

Clause 7. The compound according to any one of the preceding clauses wherein the compound of formula (I), or a pharmaceutically acceptable salt thereof, is administered for 8 weeks.

Clause 8. The compound according to any one of the preceding clauses wherein the compound of formula (I), or a pharmaceutically acceptable salt thereof, is administered in an amount of 1.7 mg/$cm^2$.

Clause 9. A compound of general formula (I)

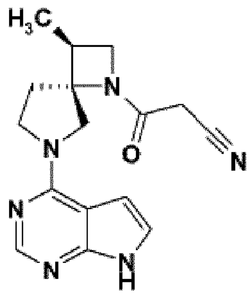

(I)

3-[(3S,4R)-3-methyl-6-(7H-pyrrolo[2,3d]pyrimidin-4-yl)-1,6-diazaspiro[3,4]octan-1-yl]-3-oxopropanenitrile, for use in the treatment of hand eczema.

Clause 10. The compound according to clause 9, for use in the treatment of chronic hand eczema.

Clause 11. The compound according to clause 9 or 10 wherein the treatment is topical treatment.

Clause 12. The compound according to any one of the preceding clauses 9-11 wherein the compound of formula (I) is administered in an ointment.

Clause 13. The compound according to any one of the preceding clauses 9-12 wherein the compound of formula (I) is administered as 30 mg/g concentration.

Clause 14. The compound according to any one of the preceding clauses 9-13 wherein the compound of formula (I) is administered as a twice daily application.

Clause 15. The compound according to any one of the preceding clauses 9-14 wherein the compound of formula (I) is administered for 8 weeks.

Clause 16. The compound according to any one of the preceding clauses 9-15 wherein the compound of formula (I) is administered in an amount of 1.7 mg/cm$^2$.

Clause 17. A use of the compound of formula (I) or a pharmaceutically acceptable salt thereof, in the manufacture of a pharmaceutical composition for the treatment of hand eczema.

Clause 18. The use according to clause 17, for the treatment of chronic hand eczema.

Clause 19. The use according to clause 17 or 18 wherein the treatment is topical treatment.

Clause 20. The use according to any one of the preceding clauses 17-19 wherein the pharmaceutical composition is an ointment.

Clause 21. The use according to any one of the preceding clauses 17-20 wherein the compound of formula (I), or a pharmaceutically acceptable salt thereof, is administered as 30 mg/g concentration.

Clause 22. The use according to any one of the preceding clauses 17-21 wherein the compound of formula (I), or a pharmaceutically acceptable salt thereof, is administered as a twice daily application.

Clause 23. The use according to any one of the preceding clauses 17-22 wherein the compound of formula (I), or a pharmaceutically acceptable salt thereof, is administered for 8 weeks.

Clause 24. The use according to any one of the preceding clauses 17-23 wherein the compound of formula (I), or a pharmaceutically acceptable salt thereof, is administered in an amount of 1.7 mg/cm$^2$.

Clause 25. A pharmaceutical composition comprising a compound of formula (I)

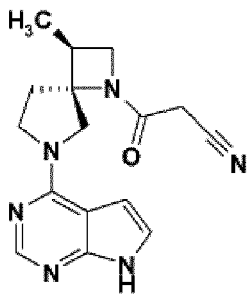

(I)

3-[(3S,4R)-3-methyl-6-(7H-pyrrolo[2,3d]pyrimidin-4-yl)-1,6-diazaspiro[3,4]octan-1-yl]-3-oxopropanenitrile, or a pharmaceutically acceptable salt thereof, for the treatment of hand eczema.

Clause 26. The pharmaceutical composition according to clause 25 for the treatment of chronic hand eczema.

Clause 27. The pharmaceutical composition according to clause 25 or 26 wherein the treatment is topical treatment.

Clause 28. The pharmaceutical composition according to any one of the preceding clauses 25-27 wherein the pharmaceutical composition is an ointment.

Clause 29. The pharmaceutical composition according to any one of the preceding clauses 25-28 wherein the compound of formula (I), or a pharmaceutically acceptable salt thereof, is administered as 30 mg/g concentration.

Clause 30. The pharmaceutical composition according to any one of the preceding clauses 25-29 wherein the

compound of formula (I), or a pharmaceutically acceptable salt thereof, is administered as a twice daily application.

Clause 31. The pharmaceutical composition according to any one of the preceding clauses 25-30 wherein the compound of formula (I), or a pharmaceutically acceptable salt thereof, is administered for 8 weeks.

Clause 32. The pharmaceutical composition according to any one of the preceding clauses 25-31 wherein the compound of formula (I), or a pharmaceutically acceptable salt thereof, is administered in an amount of 1.7 $mg/cm^2$.

Clause 33. A pharmaceutical composition comprising a compound of formula (I)

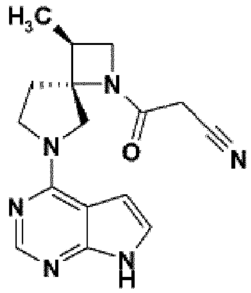

(I)

3-[(3S,4R)-3-methyl-6-(7H-pyrrolo[2,3d]pyrimidin-4-yl)-1,6-diazaspiro[3,4]octan-1-yl]-3-oxopropanenitrile, for the treatment of hand eczema.

Clause 34. The pharmaceutical composition according to clause 33 for the treatment of chronic hand eczema.

Clause 35. The pharmaceutical composition according to clause 33 or 34 wherein the treatment is topical treatment.

Clause 36. The pharmaceutical composition according to any one of the preceding clauses 33-35 wherein the pharmaceutical composition is an ointment.

Clause 37. The pharmaceutical composition according to any one of the preceding clauses 33-36 wherein the compound of formula (I) is administered as 30 mg/g concentration.

Clause 38. The pharmaceutical composition according to any one of the preceding clauses 33-37 wherein the compound of formula (I) is administered as a twice daily application.

Clause 39. The pharmaceutical composition according to any one of the preceding clauses 33-38 wherein the compound of formula (I) is administered for 8 weeks.

Clause 40. The pharmaceutical composition according to any one of the preceding clauses 33-39 wherein the compound of formula (I) is administered in an amount of 1.7 $mg/cm^2$.

Clause 41. A method for treating hand eczema in a subject in need thereof, which method comprises the step of administering to said subject a therapeutically effective amount of the compound of formula (I), or a pharmaceutically acceptable salt thereof.

Clause 42. The method according to clause 41 for treating chronic hand eczema.

Clause 43. The method according to clause 41 or 42 wherein the administration is topical.

Clause 44. The method according to any one of the preceding clauses 41-43 wherein the formulation is an ointment.

Clause 45. The method according to any one of the preceding clauses 41-44 wherein the compound of formula (I), or a pharmaceutically acceptable salt thereof is administered in a concentration of 30 mg/g.

Clause 46. The method according to any one of the preceding clauses 41-45 wherein the compound of formula (I), or a pharmaceutically acceptable salt thereof is administered as a twice daily application.

Clause 47. The method according to any one of the preceding clauses 41-46 wherein the compound of formula (I), or a pharmaceutically acceptable salt thereof is administered for 8 weeks.

Clause 48. The method according to any one of the preceding clause 41-47 wherein the compound of formula (I), or a pharmaceutically acceptable salt thereof is administered in an amount of 1.7 $mg/cm^2$.

Clause 49. A method for treating hand eczema in a subject in need thereof, which method comprises the step of administering to said subject a therapeutically effective amount of the compound of formula (I).

Clause 50. The method according to clause 49 for treating chronic hand eczema.

Clause 51. The method according to clause 49 or 50 wherein the administration is topical.

Clause 52. The method according to any one of the preceding clauses 49-51 wherein the formulation is an ointment.

Clause 53. The method according to any one of the preceding clauses 49-52 wherein the compound of formula (I) is administered in a concentration of 30 mg/g.

Clause 54. The method according to any one of the preceding clauses 49-53 wherein the compound of formula (I) is administered as a twice daily application.

Clause 55. The method according to any one of the preceding clauses 49-54 wherein the compound of formula (I) is administered for 8 weeks.

Clause 56. The method according to any one of the preceding clause 49-55 wherein the compound of formula (I) is administered in an amount of 1.7 $mg/cm^2$.

Clause 57. A therapeutic or preventive agent for hand eczema, comprising a compound of formula (I)

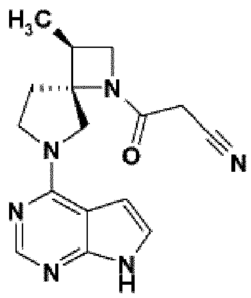

(I)

3-[(3S,4R)-3-methyl-6-(7H-pyrrolo[2,3d]pyrimidin-4-yl)-1,6-diazaspiro[3,4]octan-1-yl]-3-oxopropanenitrile, or a pharmaceutically acceptable salt thereof, as an active ingredient.

Clause 58. The therapeutic or preventive agent according to clause 57 for the treatment or prevention of chronic hand eczema.

Clause 59. The therapeutic or preventive agent according to clause 57 or 58 wherein the therapeutic or preventive agent for hand eczema is a topical agent.

Clause 60. The therapeutic or preventive agent according to any one of the preceding clauses 57-59 wherein the therapeutic or preventive agent is an ointment.

Clause 61. The therapeutic or preventive agent according to any one of the preceding clauses 57-60 wherein the compound of formula (I), or a pharmaceutically acceptable salt thereof, is administered as 30 mg/g concentration.

Clause 62. The therapeutic or preventive agent according to any one of the preceding clauses 57-61 wherein the compound of formula (I), or a pharmaceutically acceptable salt thereof, is administered as a twice daily application.

Clause 63. The therapeutic or preventive agent according to any one of the preceding clauses 57-62 wherein the compound of formula (I), or a pharmaceutically acceptable salt thereof, is administered for 8 weeks.

Clause 64. The therapeutic or preventive agent according to any one of the preceding clauses 57-63 wherein the compound of formula (I), or a pharmaceutically acceptable salt thereof, is administered in an amount of 1.7 $mg/cm^2$.

## Claims

1. A compound of general formula (I)

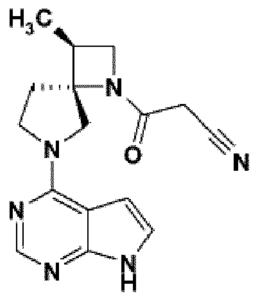

(I) ,

3-[(3S,4R)-3-methyl-6-(7H-pyrrolo[2,3d]pyrimidin-4-yl)-1,6-diazaspiro[3,4]octan-1-yl]-3-oxopropanenitrile, or a pharmaceutically acceptable salt thereof for use in the treatment of hand eczema.

2. The compound according to claim 1 wherein the treatment is topical treatment.

3. The compound according to claim 1 or 2 wherein the compound of formula (I) or a pharmaceutically acceptable salt thereof is administered in an ointment.

4. The compound according to any one of the preceding claims wherein the compound of formula (I), or a pharmaceutically acceptable salt thereof is administered in a concentration of 30 mg/g.

5. The compound according to any one of the preceding claims wherein the compound of formula (I), or a pharmaceutically acceptable salt thereof is administered as a twice daily application for 8 weeks.

6. The compound according to any one of the preceding claims wherein the compound of formula (I), or a pharmaceutically acceptable salt thereof is administered in an amount of 1.7 $mg/cm^2$.

7. A pharmaceutical composition comprising a compound of formula (I)

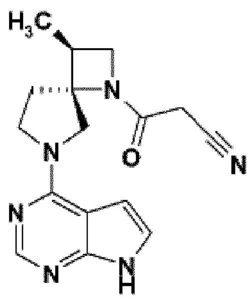

(I)

3-[(3S,4R)-3-methyl-6-(7H-pyrrolo[2,3d]pyrimidin-4-yl)-1,6-diazaspiro[3,4]octan-1-yl]-3-oxopropanenitrile, or a pharmaceutically acceptable salt thereof, for the treatment of hand eczema.

8. The pharmaceutical composition according to claim 7 wherein the treatment is topical treatment.

9. The pharmaceutical composition according to claim 7 or 8 wherein the pharmaceutical composition is an ointment.

**10.** The pharmaceutical composition according to any one of the preceding claims 7-9 wherein the compound of formula (I), or a pharmaceutically acceptable salt thereof is administered in a concentration of 30 mg/g.

**11.** The pharmaceutical composition according to any one of the preceding claims 7-10 wherein the compound of formula (I), or a pharmaceutically acceptable salt thereof is administered as a twice daily application for 8 weeks.

**12.** The pharmaceutical composition according to any one of the preceding claims 7-11 wherein the compound of formula (I), or a pharmaceutically acceptable salt thereof is administered in an amount of 1.7 $mg/cm^2$.

**13.** A method for treating hand eczema in a subject in need thereof, which method comprises the step of administering to said subject a therapeutically effective amount of the compound of formula (I)

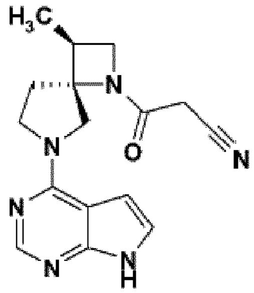

(I) ,

3-[(3S,4R)-3-methyl-6-(7H-pyrrolo[2,3d]pyrimidin-4-yl)-1,6-diazaspiro[3,4]octan-1-yl]-3-oxopropanenitrile, or a pharmaceutically acceptable salt thereof.

**14.** The method according to claim 13 wherein the administration is topical.

**15.** The method according to claim 13 or 14 wherein the topical administration is an ointment.

Fig 1

Treatment success according to PGA by visit (observed cases): full analysis set

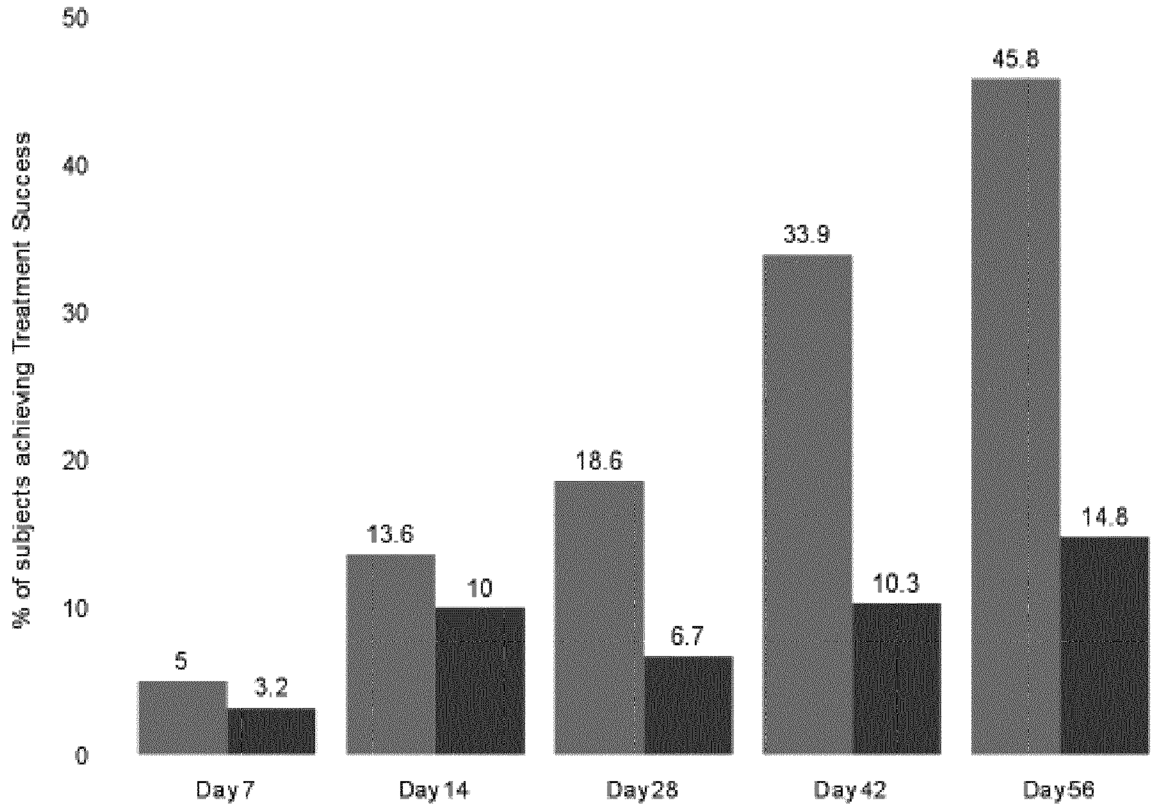


Treatment:

Grey colour: The compound of formula (I)

Black colour: Vehicle

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011013785 A
- EP 2813228 A1